# EUROPEAN PATENT APPLICATION

(11) **EP 2 868 312 A1**
(43) Date of publication of application: **06.05.2015**
(21) Application number: 13810704.0
(22) Date of filing: 28.05.2013
(51) Int. Cl.: A61K 8/49, A61K 8/19, A61K 8/35, A61K 8/368, A61Q 5/10

(54) **HAIR-DYE COMPOSITION**

(30) Priority: 27.06.2012 JP 2012144160
(71) Applicant: FUJI-FILM Corporation, Minato-ku Tokyo 106-8620 (JP)
(72) Inventor: AIMI, Makiko, Ashigarakami-gun Kanagawa 258-8577 (JP); MIKOSHIBA, Kazufumi, Tokorozawa-shi Saitama 359-0024 (JP); YAMADA, Minoru, Tokorozawa-shi Saitama 359-0024 (JP)
(74) Representative: Morpeth, Fraser Forrest
(86) International application number: PCT/JP2013/064776
(87) International publication number: WO 2014/002670

(57) **Abstract**

A hair dye composition, including a first agent and a second agent, (1) the first agent including: (a) at least one selected from the group consisting of tannic acid, gallic acid, and a gallic acid derivative; and (b) 0.5% by mass to 10% by mass of ammonium hydrogen carbonate with respect to the total mass of the first agent, and (2) the second agent including an iron salt.

## Description

### Technical Field

The invention relates to a hair dye composition.

### Background Art

Hair dyes are generally classified into four types, namely, oxidative dyes, ionic dyes, temporary dyes, and other dyes.

Among these hair dyes, an oxidative dye, which is most widely-used at present, is sometimes referred to as a permanent dye. The oxidative dye is mainly constituted with a paraphenylenediamine or a p-aminophenol, from which an active reaction intermediate is formed when oxidized with hydrogen peroxide. The activated reaction intermediate then reacts with a dye coupler molecule in hair, and it changes to a shampoo-resistant dye. However, the oxidative dye may cause damage to hair or contact dermatitis. Furthermore, the oxidative dye may remain in the human body for a long period of time, and may act as a mutagenic or carcinogenic substance.

An ionic dye is sometimes referred to as a semi-permanent dye, and it does not damage hair. However, the ionic dye may cause skin coloration upon dyeing hair. Furthermore, the ionic dye may be washed off from hair as a result of four to ten shampooing operations.

A temporary dye does not damage hair, and skin coloration can be removed by washing. However, the temporary dye is washed off from hair as a result of a single shampooing operation.

As another dye, there has been proposed a hair dye that contains a polyvalent phenol that is excellent in terms of safety and an iron salt (for example, see Japanese Patent Application Laid-Open (JP-A) No. 2008-273969 and JP-ANo. 2011-126845).

Examples of the polyvalent phenol include tannic acid, gallic acid, a gallic acid derivative, and pyrogallol.

### SUMMARY OF INVENTION

### Technical Problem

However, while tannic acid, gallic acid, and a gallic acid derivative are excellent in terms of safety, it is sometimes difficult to handle them for hair dyes since they have a higher molecular weight compared to pyrogallol, have insufficient hair dyeing property, and are easily degraded.

Furthermore, previous non-oxidative hair dyes have required a long dyeing time, and their hair dyeing property and color tone have not been satisfactory.

Under such circumstances, the development of a non-oxidative hair dye that contains tannic acid, gallic acid, and/or a gallic acid derivative as well as an iron salt and has favorable hair dyeing property and formulation stability has been demanded.

An object of the invention is to provide a hair dye composition having favorable hair dyeing property, a hair dye set including the hair dye composition, and a hair dyeing method using the hair dye composition.

### Solution to Problem

Means for solving the problems is as follows.
<1> A hair dye composition, comprising a first agent and a second agent,
   (1) the first agent comprising:
      (a) at least one selected from the group consisting of tannic acid, gallic acid, and a gallic acid derivative; and
      (b) 0.5% by mass to 10% by mass of ammonium hydrogen carbonate with respect to the total mass of the first agent, and
   (2) the second agent comprising an iron salt.
<2> The hair dye composition according to <1>, wherein the first agent further comprises an ultraviolet absorber having a 2-hydroxybenzophenone skeleton.
<3> The hair dye composition according to <1> or <2>, wherein the first agent further comprises at least one selected from the group consisting of salicylic acid and a salicylic acid derivative.
<4> The hair dye composition according to any one of <1> to <3>, wherein the iron salt is at least one selected from the group consisting of ferrous sulfate, ferrous chloride, ferrous acetate, ferric sulfate, ferric chloride, and ferric acetate.
<5> The hair dye composition according to any one of <1> to <4>, wherein a content of (a) the at least one selected from the group consisting of tannic acid, gallic acid, and a gallic acid derivative with respect to the total mass of the first agent is from 0.01% by mass to 3% by mass, and wherein a content of (2) the iron salt with respect to the total mass of the second agent is from 0.5% by mass to 10% by mass.
<6> The hair dye composition according to any one of <1> to <5>, wherein the first agent further comprises an antioxidant.
<7> A hair dye set, comprising the hair dye composition according to any one of <1> to <6>.
<8> A hair dyeing method, comprising applying the hair dye composition according to any one of <1> to <6> to hair.

### Advantageous Effects of Invention

According to the invention, a hair dye composition having favorable hair dyeing property, a hair dye set including the hair dye composition, and a hair dyeing method using the hair dye composition can be provided.

### DESCRIPTION OF EMBODIMENTS

Hereinbelow, the invention is described in detail.

In this specification, each numerical range specified using "(from) ... to ..." represents a range including the numerical values noted before and after "to" as the minimum value and the maximum value, respectively.

The term "step" as used herein indicates not only a separate step but also a step that is not clearly distinguished from other step as long as the desired effect of the step is obtained therefrom.

Furthermore, in this specification, in the reference to the amount of each component in the composition, when the composition includes plural substances corresponding to each component, the amount of the each component means the total amount of the plural substances unless otherwise specified.

### Hair Dye Pomposition

The hair dye composition according to the invention is a two-agent hair dye composition that includes a first agent and a second agent, in which (1) the first agent includes (a) at least one selected from the group consisting of tannic acid, gallic acid, and a gallic acid derivative, and (b) 0.5% by mass to 10% by mass of ammonium hydrogen carbonate with respect to the total mass of the first agent; and (2) the second agent includes an iron salt.

Hereinbelow, each of the components in the hair dye composition according to the invention is described.

### First Agent

The first agent includes (a) at least one selected from the group consisting of tannic acid, gallic acid, and a gallic acid derivative, and (b) 0.5% by mass to 10% by mass of ammonium hydrogen carbonate with respect to the total mass of the first agent.

### (a) Tannic Acid, Gallic Acid, Gallic Acid Derivative

The first agent includes (a) at least one selected from the group consisting of tannic acid, gallic acid, and a gallic acid derivative.

Examples of the tannic acid include a hydrolyzable tannin and a condensed tannin.

The hydrolyzable tannin is a compound in which an aromatic compound such as gallic acid or ellagic acid is esterified to sugar such as glucose.

Specific examples of the hydrolyzable tannin include tannic acid derived from a plant such Rhus chinensis, gallnut, sumac, tara, chestnut, myrobalan, oak, divi-divi, algarobilla, or Geranium thunbergii.

The condensed tannin is a compound in which a compound having a flavanol skeleton is polymerized.

Specific examples of the condensed tannin include tannic acid derived from a plant such as gambier, quebracho, mimosa, mangrove, hemlock, spruce, Burma cutch, oak bark, or persimmon.

In the hair dye composition according to the invention, the tannic acid may be used singly, or in combination of two or more kinds thereof.

The tannic acid may be chemically synthesized according to a known method or may be isolated from the above-described plant. The tannic acid may be prepared by further performing chemical synthesis on tannic acid isolated from the above-described plant. Furthermore, an extract containing tannic acid obtained from the above-described plant may be directly used.

The commercially available product may be used as the tannic acid. Examples thereof include LEXSOD P (trade name, manufactured by ICHIMARU PHARCOS Co., Ltd.) and G TANNIC ACID (trade name, manufactured by DSP GOKYO FOOD & CHEMICAL Co., Ltd).

Gallic acid (also known as 3,4,5-trihydroxybenzoic acid, CAS registry No. 149-91-7) is an aromatic carboxylic acid represented by the chemical formula of C₆H₂(OH)₃CO₂H and having a molecular weight of 170.12.

The gallic acid may be chemically synthesized according to a known method or may be isolated from a leguminous plant or an anacardiaceous plant. The gallic acid may be prepared by further performing chemical synthesis on gallic acid isolated from the above-described plant. Furthermore, an extract containing gallic acid obtained from the above-described plant may be directly used.

The commercially available product may be used as the gallic acid. Examples thereof include purified gallic acid (manufactured by DSP GOKYO FOOD & CHEMICAL Co., Ltd).

Examples of gallic acid derivative include an alkyl ester of gallic acid, 2,3,4-trihydroxybenzophenone (3THBP), and 2,3,4,4'-tetrahydroxybenzophenone (4THBP).

Examples of the alkyl ester of gallic acid include one containing in the molecule thereof a linear or branched-alkyl group having from 1 to 10 carbon atoms, preferably from 2 to 5 carbon atoms. Specific examples thereof include methyl gallate, ethyl gallate, and propyl gallate.

The gallic acid derivative used in the hair dye composition according to the invention may be any type of gallic acid derivative. From the view point of hair dyeing property, a gallic acid derivative having a ClogP value of from 1 to 5 can be preferably used. It is noted that logP is a parameter of hydrophobicity and indicates the octanol/water partition coefficient. As a result of the development of the recent computational chemistry, it became possible to obtain a logP value by calculation based on computer. The value thus obtained is referred to as ClogP. In the invention, the value calculated using ChemDrawPro (manufactured by CambridgeSoft) is used as the ClogP value, but the ClogP value is not limited to this.

The gallic acid derivative may be used singly, or in combination of two or more kinds thereof in the hair dye composition according to the invention.

The gallic acid derivative may be chemically synthesized according to a known method or may be isolated from a plant such as Rhus chinensis. The gallic acid derivative may be prepared by further performing chemical synthesis on gallic acid derivative isolated from the above-described plant. Furthermore, an extract containing a gallic acid derivative obtained from the above-described plant may be directly used.

The commercially available product may be used as the gallic acid derivative. Examples thereof include TEAVIGO (DSM Nutrition Japan KK) and propyl gallate (manufactured by DSP GOKYO FOOD & CHEMICAL Co., Ltd).

The content of (a) the at least one selected from the group consisting of tannic acid, gallic acid, and a gallic acid derivative is not particularly limited, and preferably from 0.01% by mass to 3% by mass with respect to the total mass of the first agent from the viewpoint of usability.

In the hair dye composition according to the invention, any of (a) tannic acid, gallic acid, and a gallic acid derivative may be used singly, or in combination of two or more kinds thereof.

### (b) Ammonium Hydrogen Carbonate

The first agent includes 0.5% by mass to 10% by mass of ammonium hydrogen carbonate with respect to the total mass of the first agent.

Ammonium hydrogen carbonate (CAS registry No. 1066-33-7) is represented by the chemical formula of NH₄HCO₃.

Ammonium hydrogen carbonate can be chemically synthesized according to a known method. The commercially available product may be used as the ammonium hydrogen carbonate. Examples thereof include ammonium hydrogen carbonate (manufactured by Ube Chemical).

The content of the ammonium hydrogen carbonate is preferably from 1% by mass to 8% by mass and more preferably from 1% by mass to 4% by mass, from the viewpoint of hair dyeing property, formulation stability, and damage to hair.

### Ultraviolet Absorber

In the invention, the first agent may further include an ultraviolet absorber having a 2-hydroxybenzophenone skeleton.

The ultraviolet absorber having a 2-hydroxybenzophenone skeleton is preferably a compound having a 2-hydroxybenzophenone skeleton represented by the following Formula (1).

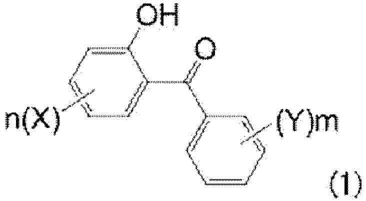

In Formula (1), each of X and Y independently represents a hydroxyl group, an alkoxy group having from 1 to 16 carbon atoms, an alkyl group having from 1 to 16 carbon atoms, a sulfo group, a carboxy group, or a halogen atom. Each of n and m independently represents an integer of 1 to 5.

Specific examples of the ultraviolet absorber having a 2-hydroxybenzophenone skeleton include, but are not limited to, oxybenzone-1 (2,4-dihydroxybenzophenone), oxybenzone-3 (2-hydroxy-4-methoxybenzophenone), oxybenzone-4 (2-hydroxy-4-methoxybenzophenone sulfonic acid), oxybenzone-6 (2,2'-dihydroxy-4,4'-dimethoxybenzophenone), tetrahydroxybenzophenone, oxybenzone-9 (2,2'-dihydroxy-4,4'-dimethoxybenzophenone disulfonic acid), 4-ethoxy-2-hydroxybenzophenone, 4-(2-ethylhexyloxy)-2-hydroxybenzophenone, 5-amino-2-hydroxybenzophenone, 4-amino-2-hydroxybenzophenone, 4'-amino-2-hydroxybenzophenone, 2-hydroxy-5-chlorobenzophenone, 2-hydroxy-3,5-dichlorobenzophenone, 3',5-dichloro-2-hydroxy-benzophenone, 2-hydroxy-4'-methylbenzophenone, 2-hydroxy-4'-methoxybenzophenone, 2-hydroxy-5-methylbenzophenone, 2-hydroxy-4'-methylbenzophenone, and 2-hydroxy-3-tert-butylbenzophenone. Among these, oxybenzone-1, oxybenzone-3, oxybenzone-4, oxybenzone-6, and tetrahydroxybenzophenone are preferable, and oxybenzone-1, oxybenzone-3, and oxybenzone-4 are more preferable.

The content of the ultraviolet absorber having a 2-hydroxybenzophenone skeleton with respect to the total mass of the first agent may be from 0.1% by mass to 5% by mass, and preferably from 0.5% by mass to 3% by mass, from the view point of hair dyeing property and formulation stability.

In the first agent according to the invention, the ultraviolet absorber having a 2-hydroxybenzophenone skeleton may be used singly, or in combination of two or more kinds thereof.

### Salicylic Acid , Salicylic Acid Derivative

The first agent according to the invention may further include at least one selected from the group consisting of salicylic acid and a salicylic acid derivative.

Examples of the salicylic acid derivative include a salicylic acid ester and a salicylic acid salt. Examples of the salicylic acid salt include alkali metal salts of salicylic acid. Specific examples thereof include sodium salicylate.

Specific examples of the salicylic acid ester include methyl salicylate and ethyl salicylate.

In the first agent according to the invention, salicylic acid and/or the salicylic acid derivative may be used singly, or in combination of two or more kinds thereof.

The content of the salicylic acid and/or the salicylic acid derivative with respect to the total mass of the first agent may be from 0.1% by mass to 5% by mass and preferably from 0.5% by mass to 3% by mass, from the view point of hair dyeing property and formulation stability.

### Other- Component that Reacts with Iron to Form Color

In addition to (a) tannic acid, gallic acid, and/or a gallic acid derivative, the first agent according to the invention may include other component that reacts with iron to form color.

The other component that reacts with iron to form color may be a compound obtained by organic synthesis or a plant extract. The known component may be used as the other component.

Specific examples of the other component that reacts with iron to form color include logwood, hematein, catechol, phthalic acid, eugenol, isoeugenol, nicotinic-acid amide, dehydroacetic acid, pyridoxine, ellagic acid, kojic acid, maltol, ferulic acid, hinokitiol, turmeric extract, curcumin, Scutellaria root extract, onion extract, quercetin, rutin, hesperetin, hesperidin, fresh coffee bean extract, caffeic acid, chlorogenic acid, tea extract, catechin, epicatechin, lithospermi radix extract, Japanese basil extract, shisonin, grape leaf extract, grape extract, enocyanin, laccaic acid, lac, cochineal, carminic acid, elderberry, red cabbage, purple sweet potato, tamarind, kaoliang, apigeninidin, and luteolinidin. More preferably, ferulic acid, Scutellaria root extract, or quercetin may be appropriately blended.

In the first agent according to the invention, the other component that reacts with iron to form color may be used singly, or in combination of two or more kinds thereof.

### Antioxidant

The first agent according to the invention may further include an antioxidant.

The antioxidant that can be used may be one used for the usual hair dye composition, and examples thereof include a sulfite, a bisulfite, a thiosulfate, an ascorbic acid, a thioglycolic acid, a cysteine compound, and a mercapto compound More specifically, preferable examples of the antioxidant include sodium sulfite, L-ascorbic acid and a salt thereof, thioglycolic acid and a salt thereof, L-cysteine and a salt thereof, and N-acetyl-L-cysteine and a salt thereof. Among these, sodium sulfite is preferable since it contributes the stabilization of a dye precursor substance as well as improves dyeing power.

The antioxidant may be used singly, or in combination of two or more kinds thereof.

The content of the antioxidant with respect to the total mass of the first agent may be from 0.1% by mass to 10% by mass and preferably from 0.5% by mass to 5% by mass, from the view point of storage stability and usability.

### Second Agent

The second agent includes an iron salt.

Any known iron salt may be used as long as it reacts with (a) tannic acid, gallic acid, and/or the gallic acid derivative to form color and fix the dye to hair.

Examples of the iron salt include ferrous sulfate, ferrous chloride, ferrous acetate, ferrous phosphate, ferrous oxalate, ferric sulfate, ferric chloride, and ferric acetate. Among these, from the viewpoint of color forming property, ferrous sulfate, ferrous chloride, ferrous acetate, ferric sulfate, ferric chloride, and ferric acetate are preferable, and ferrous sulfate, ferrous chloride, and ferric chloride are more preferable.

In the second agent according to the invention, the iron salt may be used singly, or in combination of two or more kinds thereof.

The content of the iron salt is not particularly limited. The content of the iron salt with respect to the total mass of the second agent is preferably from 0.5% by mass to 10% by mass, more preferably from 1% by mass to 6% by mass, and still more preferably from 2% by mass to 5% by mass, from the viewpoint of color forming property.

In addition to the iron salt, the second agent may further include the antioxidant described above with respect to the first agent.

In the second agent, the antioxidant included is preferably L-ascorbic acid, a salt of L-ascorbic acid, or sodium sulfite, and more preferably L-ascorbic acid or a salt of L-ascorbic acid, from the view point of formulation stability and usability.

Specific examples of the salt of L-ascorbic acid include sodium L-ascorbate and calcium L-ascorbate.

In the second agent, the antioxidant may be used singly, or in combination of two or more kinds thereof.

In the second agent, the content of the antioxidant with respect to the total mass of the second agent is preferably from 0.1% by mass to 10% by mass and more preferably from 0.5% by mass to 5% by mass, from the view point of formulation stability and usability.

### Other Components

The first and/or second agent may further include various kinds of other components in addition to the above ingredients.

Examples of the other components include a base, a surfactant, a solvent, a pH adjuster, a wetting agent, a thickener, fat and oil, an organic acid, an antiseptic, perfume, a metallic smell masking agent, and a coloring agent.

Examples of the other components that may be blended as appropriate include components used for ordinary cosmetic products, such as a hair restorer/hair growth stimulant, an anti-dandruf agent, an antibacterial agent, a softener, a moisturizer, an active oxygen removing agent, an antioxidant, an antimicrobial agent, astaxanthin, silicone, mineral, a protein hydrolysate, a peptide, an amino acid, collagen, and collagen hydrolysate, within a range that does not impair the object of the invention.

The amounts of the other components may be appropriately determined within a range in which the effects of the invention are exhibited.

Examples of the base include a higher alcohol, a hydrocarbon, a fatty acid ester, a vegetable oil, and a fatty acid.

Examples of the surfactant include a polyoxyethylene alkyl ether, a polyoxyethylene, a polyoxypropylene alkyl ether, a glycerin fatty acid ester, a polyglycerin fatty acid ester, a polyethylene glycol fatty acid ester, a polyoxyethylene alkyl ether phosphate and a salt thereof, an alkylglucoside, an N-acylamino acid salt, an alkyl ether carboxylate, an alkyl sulfate, a polyoxyethylene alkyl ether sulfate, a sulfonate, an alkyl ammonium salt, and an alkyl amide propyl betaine.

Examples of the solvent include water, ethanol, isopropyl alcohol, 1,3-butylene glycol, 2-methyl-2,4-pentanediol, glycerin, and propylene glycol.

Examples of the pH adjuster include ammonia, ammonium carbonate, potassium hydroxide, sodium hydroxide, monoethanolamine, isopropanolamine, and hydrochloric acid.

Examples of the wetting agent include propylene glycol, glycerin, sorbitol, sodium pyrroridonecarboxylate, an amino acid, and a vegetable oil.

Examples of the thickener include xanthan gum, polyethylene glycol, and hydroxyethyl cellulose. In particular, preferable examples of the thickener include xanthan gum, from the viewpoint of formulation stability.

The xanthan gum is a polysaccharide obtained by carbohydrate fermentation using Xanthomonas bacteria.

The xanthan gum may further include cellulase. The xanthan gum may be a powdered xanthan gum or a granular xanthan gum.

As described above, the xanthan gum can be obtained by carbohydrate fermentation using Xanthomonas bacteria according to a conventional method. Examples of the carbohydrate include starch, glucose, and sucrose.

Examples of the Xanthomonas bacteria include Xanthomonas canpestris Xanthomonas, Xanthomonas carotate, Xanthomonas incanae, Xanthomonas begonia, Xanthomonas papavericola, Xanthomonas translucens, Xanthomonas vasculorum, and Xanthomonas hederae.

It is preferable to use sodium, potassium, or calcium as a counter ion for the carbohydrate fermentation to obtain the xanthan gum.

The purification may be conducted after the fermentation to obtain the xanthan gum.

Examples of the purification method include centrifugation, filtration, purification by enzymatic treatment, and heating and/or an organic solvent treatment.

The hair dye composition according to the invention include the first agent and the second agent.

The first agent and the second agent are simultaneously or separately mixed. The mass ratio of the mixture (first agent: second agent) is preferably in a range of from 1:0.5 to 1:2, more preferably in a range of from 1:0.8 to 1:1.2, and still more preferably 1:1.

The pH value of the first agent is preferably from pH 5 to pH 10, and more preferably from pH 6 to pH 9.

The pH value of the second agent is preferably from pH 2 to pH 6, and more preferably from pH 3 to pH 5.

### Hair Dye Set

The hair dye set according to the invention is a hair dye composition including the first agent and the second agent according to the invention.

The hair dye set according to the invention is preferably a two-agent hair dye including the first agent and the second agent. The hair dye set according to the invention is formed by combining the first agent and the second agent.

The dosage forms of the first agent and the second agent in the hair dye set are not particularly limited. Examples thereof include cream, liquid, gel, emulsion, spray, and aerosol. Among these, aerosol is preferable. The dosage forms of the first agent and the second agent may be same as or different from each other.

Each of the first agent and the second agent may further include a known component required for preparing the formulation that can be selected according to the type of the dosage form thereof.

The known component required for preparing the formulation may be appropriately selected according to the type of the dosage form thereof.

In the case of the aerosol dosage form, the aerosol can be produced by filling a pressure-resistant container with the first agent or the second agent, compressed gas, a surfactant, a thickener, and/or liquefied gas, etc. under an anaerobic atmosphere. The compressed gas used herein is preferably nitrogen gas, carbonic acid gas, argon gas, or the like. The other dosage forms can be prepared in accordance with known methods.

### Hair Dyeing Method

The hair dyeing method according to the invention includes applying to hair the hair dye composition that includes the first agent and the second agent according to the invention. Here, the above-described definitions apply to the first agent and the second agent as they are.

The preferable examples of the hair include head hair.

Examples of the method of applying the hair dye composition to hair include a method in which the first agent is applied to hair and left for a predetermined period of time, and then the second agent is applied to the hair and left for a predetermined period of time, followed by washing the first and second agents off. Other examples of the method for applying the hair dye to hair include a method in which the first agent and the second agent are simultaneously applied to hair, and then left for a predetermined period of time, followed by washing the first and second agents off.

The application of the hair dye composition to hair can be carried out in accordance with a known method. More specifically, the hair dye composition may be applied to the hair directly or using hands or a tool such as a brush. From the viewpoint of prevention of splattering and dripping of the agent, the application is preferably carried out with gloved hands.

The leaving time of the first agent and the second agent may be appropriately selected. The known method can be used as the method of washing the first and second agents off.

The amount of the hair dye composition to be applied can be appropriately selected. For example, it is preferable to apply approximately 30 g to 70 g of the first agent and approximately 30 g to 70 g of the second agent in the case of the head hair having a length of approximately 20 cm. It is more preferable to apply approximately 40 g to 60 g of the first agent and approximately 40 g to 60 g of the second agent. As an example, 50 g of the first agent and 50 g of the second agent may be applied.

### EXAMPLES

Hereinafter, the invention is described more specifically with reference to the examples, but the invention is not limited to these examples. Here, "part(s)" and "%" are based on mass unless otherwise specified.

### Examples 1 to 10 and Comparative Examples 1 to 5

Hair dye compositions having the compositions shown in the following Tables 1 to 3 were produced according to an ordinary method.

The numerical value regarding each component shown in the tables indicates % by mass with respect to the total mass of the first agent or the second agent. With regard to these compositions, hair dyeing property and the formulation stability of the first agent were evaluated based on the following methods.

### The results are shown in Tables 1 to 3.

The amounts of sodium hydroxide and hydrochloric acid are amounts necessary for adjusting the pH to the indicated value of 7.

### Hair Dyeing Method

2 g of the first agent was applied to 1 g of a goat hair bundle (product ID: BM-W-A; manufactured by Beaulax) having a length of approximately 10 cm, and then spread thereon uniformly. Subsequently, the resultant was left for the indicated period of time. Thereafter, 2 g of the second agent was applied thereto and then spread thereon uniformly, followed by leaving it for the indicated period of time.

Thereafter, the hair bundle was subjected to shampooing and rinsing treatments, and then dried with a dryer.

### Method of Evaluating Hair Dyeing Property

The color of each dyed hair bundle was measured with Chroma Meter CR200 manufactured by Minolta Corp. Hair dyeing property was evaluated based on a ΔL value from the goat hair before dyed in accordance with the following criteria.

Here, the ΔL value means the difference of the values measured with the Chroma Meter before and after hair dyeing.

### Evaluation Criteria

A: ΔL value ≤ 25 (goat hair is found colored very well by visual observation)
B: 25 < ΔL value ≤ 35 (goat hair is found colored well by visual observation)
C: 35 < ΔL value ≤ 45 (goat hair is found colored by visual observation)
D: 45 < ΔL value (goat hair is found hardly colored by visual observation)

### Formulation Stability

The prepared first agent was stored in a thermostat at 40°C for 1 month, and then the viscosity of the resultant was compared with that of the first agent immediately after the preparation. The viscosity was measured using a B-type viscometer under the condition of 6 rpm at 25°C. At the same time, the occurrence of separation was visually confirmed.

### Evaluation Criteria

A: change in the viscosity was within 10000 mPas, and no separation was observed.
B: change in the viscosity exceeded 10000 mPas, and/or separation was observed.

**Table 1**

| | | Example 1 | Example 2 | Example 3 | Example 4 | Example 5 |
|---|---|---|---|---|---|---|
| First agent | Tannic acid | 1.0 | 1.0 | 1.0 | 1.0 | 1.0 |
| | Oxybenzone-4 | 1.0 | 1.0 | 1.0 | 1.0 | 1.0 |
| | Ammonium hydrogen carbonate | 1.0 | 2.0 | 4.0 | 2.0 | 2.0 |
| | Benzyl alcohol | 3.0 | 3.0 | 3.0 | 3.0 | 3.0 |
| | Ethanol | 2.0 | 2.0 | 2.0 | 2.0 | 2.0 |
| | 1,3-Butylene glycol | 2.0 | 2.0 | 2.0 | 2.0 | 2.0 |
| | Xanthan gum | 0.5 | 0.5 | 0.5 | 0.5 | 0.5 |
| | Acrylic acid-methacrylic acid copolymer | 1.5 | 1.5 | 1.5 | 1.5 | 1.5 |
| | Sodium sulfite | 2.0 | 2.0 | 2.0 | 2.0 | 2.0 |
| | Potassium hydroxide | q. s. | q. s. | q. s. | q. s. | q. s. |
| | Hydrochloric acid | q. s. | q. s. | q. s. | q. s. | q. s. |
| | Purified water | Balance | Balance | Balance | Balance | Balance |
| | pH | 7.2 | 7.2 | 7.2 | 7.2 | 7.2 |
| | Leaving time (minutes) | 20 | 20 | 20 | 20 | 20 |
| Second agent | Ferrous sulfate | 2.0 | 2.0 | 2.0 | - | 2.0 |
| | Ferrous chloride | - | - | - | 2.0 | - |
| | L-Ascorbic acid | q. s. | q. s. | q. s. | q. s. | q. s. |
| | Sodium L-ascorbate | q. s. | q. s. | q. s. | q. s. | q. s. |
| | Phenoxyethanol | 0.3 | 0.3 | 0.3 | 0.3 | 0.3 |
| | Xanthan gum | 1.0 | 1.0 | 1.0 | 1.0 | 1.0 |
| | Purified water | Balance | Balance | Balance | Balance | Balance |
| | Leaving time (minutes) | 10 | 10 | 10 | 10 | 10 |
| Hair dyeing property | | B | A | A | A | A |
| Formulation stability | | A | A | A | A | A |

| | | | | | | |
|---|---|---|---|---|---|---|
| * q. s.: quantum sufficiat (appropriate amount) | | | | | | |

**Table 2**

| | | Example 6 | Example 7 | Example 8 | Example 9 | Example 10 |
|---|---|---|---|---|---|---|
| First agent | Tannic acid | 0.8 | 0.5 | 0.5 | 0.3 | 0.3 |
| | Oxybenzone-4 | 2.0 | 2.0 | - | 2.0 | 2.0 |
| | Oxybenzone-3 | - | - | 1.5 | - | - |
| | Salicylic acid | - | 1.0 | 1.0 | 0.5 | 0.5 |
| | Ammonium hydrogen carbonate | 2.0 | 2.0 | 2.0 | 2.0 | 2.0 |
| | Benzyl alcohol | 3.0 | 3.0 | 3.0 | 3.0 | 3.0 |
| | Ethanol | 2.0 | 2.0 | 2.0 | 2.0 | 2.0 |
| | 1,3-Butylene glycol | 2.0 | 2.0 | 2.0 | 2.0 | 2.0 |
| | Xanthan gum | 0.5 | 0.5 | 0.5 | 0.5 | 0.5 |
| | Acrylic acid-methacrylic acid copolymer | 1.5 | 1.5 | 1.5 | 1.5 | 1.5 |
| | Sodium sulfite | 2.0 | 2.0 | 2.0 | 2.0 | 2.0 |
| | Potassium hydroxide | q. s. | q. s. | q. s. | q. s. | q. s. |
| | Hydrochloric acid | q.s. | q.s. | q.s. | q.s. | q.s. |
| | Purified water | Balance | Balance | Balance | Balance | Balance |
| | pH | 7.2 | 7.2 | 7.2 | 7.2 | 7.2 |
| | Leaving time (minutes) | 20 | 20 | 20 | 20 | 20 |
| Second agent | Ferrous sulfate | 2.0 | 2.0 | 2.0 | 2.0 | - |
| | Ferrous chloride | - | - | - | - | 2.0 |
| | L-Ascorbic acid | q. s. | q. s. | q. s. | q. s. | q. s. |
| | Sodium L-ascorbate | q.s. | q.s. | q.s. | q.s. | q.s. |
| | Phenoxyethanol | 0.3 | 0.3 | 0.3 | 0.3 | 0.3 |
| | Xanthan gum | 1.0 | 1.0 | 1.0 | 1.0 | 1.0 |
| | Purified water | Balance | Balance | Balance | Balance | Balance |
| | Leaving time (minutes) | 10 | 10 | 10 | 10 | 10 |
| Hair dyeing property | | A | B | B | B | B |
| Formulation stability | | A | A | A | A | A |

**Table 3**

| | | Comparative Example 1 | Comparative Example 2 | Comparative Example 3 | Comparative Example 4 | Comparative Example 5 |
|---|---|---|---|---|---|---|
| First agent | Tannic acid | 1.0 | 1.0 | 1.0 | 1.0 | 1.0 |
| | Oxybenzone-4 | 1.0 | 1.0 | 1.0 | 1.0 | 1.0 |
| | Potassium hydrogen carbonate | - | 2.0 | - | - | - |
| | Urea | - | - | 2.0 | 2.0 | 4.0 |
| | Benzyl alcohol | 3.0 | 3.0 | 3.0 | 3.0 | 3.0 |
| | Ethanol | 2.0 | 2.0 | 2.0 | 2.0 | 2.0 |
| | 1,3-Butylene glycol | 2.0 | 2.0 | 2.0 | 2.0 | 2.0 |
| | Xanthan gum | 0.5 | 0.5 | 0.5 | 0.5 | 0.5 |
| | Acrylic acid-methacrylic acid copolymer | 1.5 | 1.5 | 1.5 | 1.5 | 1.5 |
| | Sodium sulfite | 2.0 | 2.0 | 2.0 | 2.0 | 2.0 |
| | Potassium hydroxide | q. s. | q. s. | q. s. | q. s. | q. s. |
| | Hydrochloric acid | q. s. | q. s. | q. s. | q. s. | q. s. |
| | Purified water | Balance | Balance | Balance | Balance | Balance |
| | pH | 7.2 | 7.2 | 7.2 | 7.2 | 7.2 |
| | Leaving time (minutes) | 20 | 20 | 20 | 20 | 20 |
| Second agent | Ferrous sulfate | 2.0 | 2.0 | 2.0 | - | 2.0 |
| | Ferrous chloride | - | - | - | 2.0 | - |
| | L-Ascorbic acid | q. s. | q. s. | q. s. | q. s. | q. s. |
| | Sodium L-ascorbate | q. s. | q. s. | q. s. | q. s. | q. s. |
| | Phenoxyethanol | 0.3 | 0.3 | 0.3 | 0.3 | 0.3 |
| | Xanthan gum | 1.0 | 1.0 | 1.0 | 1.0 | 1.0 |
| | Purified water | Balance | Balance | Balance | Balance | Balance |
| | Leaving time (minutes) | 10 | 10 | 10 | 10 | 10 |
| Hair dyeing property | | C | C | c | C | c |
| Formulation stability | | A | C | A | A | C |

These results have clarified that the hair dye composition according to the invention has favorable hair dyeing property and formulation stability (temporal stability and/or thermal stability).

The disclosure of Japanese Patent Application No. 2012-144160 filed on June 27, 2012, is incorporated herein by reference in its entirety.

All publications, patent applications, and technical standards mentioned in this specification are herein incorporated by reference to the same extent as if each individual publication, patent application, or technical standard was specifically and individually indicated to be incorporated by reference.

## Claims

1. A hair dye composition, comprising a first agent and a second agent,
(1) the first agent comprising:
(a) at least one selected from the group consisting of tannic acid, gallic acid, and a gallic acid derivative; and
(b) 0.5% by mass to 10% by mass of ammonium hydrogen carbonate with respect to the total mass of the first agent, and
(2) the second agent comprising an iron salt.

2. The hair dye composition according to claim 1, wherein the first agent further comprises an ultraviolet absorber having a 2-hydroxybenzophenone skeleton.

3. The hair dye composition according to claim 1 or 2, wherein the first agent further comprises at least one selected from the group consisting of salicylic acid and a salicylic acid derivative.

4. The hair dye composition according to any one of claims 1 to 3, wherein the iron salt is at least one selected from the group consisting of ferrous sulfate, ferrous chloride, ferrous acetate, ferric sulfate, ferric chloride, and ferric acetate.

5. The hair dye composition according to any one of claims 1 to 4, wherein a content of (a) the at least one selected from the group consisting of tannic acid, gallic acid, and a gallic acid derivative with respect to the total mass of the first agent is from 0.01% by mass to 3% by mass, and wherein a content of (2) the iron salt with respect to the total mass of the second agent is from 0.5% by mass to 10% by mass.

6. The hair dye composition according to any one of claims 1 to 5, wherein the first agent further comprises an antioxidant.

7. A hair dye set, comprising the hair dye composition according to any one of claims 1 to 6.

8. A hair dyeing method, comprising applying the hair dye composition according to any one of claims 1 to 6 to hair.
